# EUROPEAN PATENT APPLICATION

(11) **EP 2 728 333 A1**
(43) Date of publication of application: **07.05.2014**
(21) Application number: 12804898.0
(22) Date of filing: 14.06.2012
(51) Int. Cl.: G01N 1/10, G01N 1/00

(54) **SAMPLING DEVICE**

(30) Priority: 28.06.2011 JP 2011142692; 26.10.2011 JP 2011234845
(71) Applicant: Toray Medical Co., Ltd., Urayasu-shi, Chiba 279-8555 (JP)
(72) Inventor: UCHIYAMA Toshiyuki, Numazu-shi Shizuoka 410-0001 (JP)
(74) Representative: Prüfer & Partner GbR European Patent Attorneys
(86) International application number: PCT/JP2012/065199
(87) International publication number: WO 2013/002032

(57) **Abstract**

A sampling device provided with: a housing having a main flow channel that comprises a liquid inflow channel and a liquid outflow channel, and a sampling flow channel that branches from a branching part provided between the liquid inflow channel and the liquid outflow channel, one end of the sampling channel being formed as an open end; a cap for covering the open end part of the sampling flow channel, the cap being detachable from the housing; and a valve provided in an urged state so as to cut off the sampling flow channel from the main flow channel, the sampling flow channel and the main flow channel being no longer cut off when the cap is attached to the housing or when the cap is removed from the housing and a liquid sampling means is attached to the open end part of the sampling channel. The sampling device is characterized in that a venturi unit is provided to the liquid outflow channel; and, when the cap is attached to the housing, a bypass channel for interconnecting the inside of the cap and a throat part of the venturi unit is formed. This sampling device prevents liquid from accumulating in the sampling flow channel and allows the sampling region and the surrounding area to be kept at an optimal degree of cleanliness.

## Description

### Technical Field of the Invention

The present invention relates to a sampling device and, more specifically, relates to a sampling device in which the sampling region is prevented from becoming polluted.

### Background Art of the Invention

Generally, in a system for handling liquids in which the appropriate composition and the secure degree of cleanliness should be maintained, it is necessary to perform the sampling inspection (the sampling) of liquids frequently for the purposes of inspection, analysis, etc. As a method for carrying out such sampling, for example, Patent Document 1 discloses a technology in which valves are provided to inlet and outlet channels branching from a main channel and in which a sampling channel connecting between the inlet channel and the outlet channel is provided so that it becomes possible to sample a part of fluid from the main channel.

Furthermore, Patent Document 2 discloses a technology for sampling a fluid, such as liquid for dialysis, by piercing an elastic member incorporated in an component of a sampling port with an injector.

However, as the demand for cleanliness increases, several concerns are rising: for example, propagation of bacteria at a portion where a syringe needle has been pierced; generation of air bubbles or retention of liquid in a sampling channel; and contamination of sampling site (a portion and its surroundings where a liquid sampling means such as a syringe is inserted) caused by a manipulation of sampling.

For this reason, a "sampling port" has been disclosed that can perform sampling without syringe, and that can prevent contamination of sampling site by means of a detachable/attachable cap and can also prevent propagation of bacteria by soaking the sampling site and its surroundings in liquid when the sampling is not performed.

### Prior art documents

### Patent document

Patent document 1: JP-A-H7-128204
Patent document 2: JP-A-H11-169456
Patent document 3: JP-B-4352368
Patent document 4: JP-A-2009-207706

### Summary of the Invention

### Problems to be solved by the Invention

However, in the above-described sampling ports in Patent Document 3 and 4, although a circulating flow that starts from a main flow channel and comes back to the main flow channel again via a sampling site and its surroundings is generated so as to clean the sampling site when the sampling is not performed, the sampling flow channel is narrow, and the shape of the sampling site and its surroundings becomes complicated. As a consequence, there is a fear that it may be difficult for the liquid to enter the sampling flow channel from the main flow channel, and it is difficult to generate a desired circulating flow that streams from the main flow channel to the main flow channel via the sampling flow channel without stagnancy.

In addition, it is difficult to secure a certain stable flow in the sampling flow channel, because the liquid flow in the sampling flow channel is susceptible to various conditions including the volume of the liquid flow, the installation conditions of the sampling port, the orientation of the sampling port (e.g. upward orientation, landscape orientation or downward orientation), and the fluctuation of fluid pressure.

Accordingly, the object of the present invention is to provide a sampling device that can prevent retention of fluid in the sampling flow channel and can maintain the degree of cleanliness in a sampling site and its surroundings appropriately.

### Means for solving the Problems

In order to solve the above-described problem, a sampling device according to the present invention is a sampling device comprising:
a housing having a main flow channel including a liquid inflow channel and a liquid outflow channel, said housing further having a sampling flow channel branching from a branching part provided between said liquid inflow channel and said liquid outflow channel, one end of said sampling channel being formed as an open end;
a cap for covering said open end part of said sampling flow channel, said cap being detachable from and attachable to said housing; and
a valve provided in an urged state so as to interrupt said sampling flow channel from said main flow channel, said valve being configured to release the interruption of said sampling flow channel from said main flow channel when attaching said cap to said housing or when attaching a liquid sampling means to said open end part of said sampling channel subsequent to detaching said cap from said housing;
wherein a venturi portion is provided to said liquid outflow channel; and
wherein said sampling device is provided with a bypass channel being configured to connect an inner space of said cap to said throat part of said venturi portion when said cap is attached to said housing.

In the sampling device according to the present invention, when liquid flows through the main flow channel, due to liquid acceleration the pressure decreases in the throat part, which is disposed in the venturi portion and has a reduced cross-sectional area of flow, while liquid passes through the venturi portion provided in the liquid outflow channel. When the cap is attached to the housing under such a situation, the blockage of the sampling flow channel caused by the valve member is cleared, a part of liquid enters sampling flow channel, passes from the opening end of the sampling flow channel through the bypass channel via an inner space of the cap, and joins the rest of liquid that flows through the main channel at the throat part of the venturi portion. As described above, because there occurs a relatively negative pressure caused by the pressure reduction at the throat part of the venturi portion, a continuous flow that tends to draw the liquid from the bypass channel into the venturi portion occurs in a path from the sampling flow channel to the throat part of the venturi portion via the bypass channel even when the sampling flow channel is formed into a narrow intricate shape. As a result, the propagation of bacteria and the accumulation of ingredients in liquid are prevented in this path, and the degree of cleanliness is maintained appropriately.

On the other hand, when the cap is detached from the housing, the sampling flow channel is blocked by the valve member that is urged so as to shut the sampling flow channel from the main flow channel, and therefore liquid leakage from the opening end of the sampling flow channel is prevented. And when a liquid sampling means is attached to the opening end of the sampling flow channel, the blockage of the sampling channel caused by the valve member is cleared, and the sampling is performed that a part of liquid flowing through the main channel enters the liquid sampling means via the sampling flow channel. When the liquid sampling means is detached from the opening end of the sampling flow channel after the sampling is finished, the sampling flow channel is shut by means of the valve member once again and liquid leakage is prevented. Therefore, according to the sampling device of the present invention, it becomes possible to maintain the degree of cleanliness of the sampling flow channel in normal times by attaching the cap to the housing and letting liquid flow continuously in the sampling flow channel. Furthermore, even in a case where the cap is detached temporarily in order to perform sampling, liquid leakage from the sampling flow channel is prevented and therefore it becomes possible to expedite the sampling process.

In the sampling device according to the present invention, the pressure difference between the throat part and the sampling flow channel occurs even when the liquid pressure flowing through the flow channel is low, does not depend upon the flow direction, and is hardly affected by the conditions of installation of the sampling device, the changes of installation position, the amount of liquid flow and the fluctuations in pressure, etc. Therefore, it becomes possible to achieve stable liquid flow that is not affected by the installation conditions and the changes of operation conditions. Additionally, because the stable liquid flow can be achieved by means of a simplified structure without auxiliary power source, it becomes possible to avoid increasing in size and complexity and to prevent the manufacturing cost from increasing.

Moreover, the sampling device according to the present invention functions effectively, not only in the normal operation, but also in the case of cleansing wherein a cleansing liquid flows through the entire liquid processing system including the sampling device. By performing the above-described cleansing process under the situation where the cap is equipped with the housing of the sampling device, part of the cleansing liquid enters the sampling flow channel and cleanses the sampling flow channel, the inner space of the cap and the bypass channel. Even when the cleansing liquid flows in main flow channel 3 and sampling flow channel 9, a flow of the cleansing liquid occurs in a flow path from the sampling flow channel to the throat part of the venturi portion via the bypass channel by virtue of the pressure difference. Therefore, it becomes possible to cleanse the sampling flow channel quickly and efficiently, reduce the cleansing time, and improve the degree of cleanliness. In the case of disinfection as well wherein a disinfectant instead of the cleansing liquid flows through the entire liquid processing system, these advantages can be gained in like manner.

The structure of the above-described bypass channel is not limited in particular. For example, the bypass channel may be formed inside the housing and may be formed outside the housing. When the bypass channel is formed inside the housing, it is preferred that the bypass channel is provided with a valve member that shuts the bypass channel by detaching the cap from the housing and stops shutting the bypass channel by attaching the cap to the housing. According to such a structure, the liquid leakage from the bypass channel and the air contamination from the bypass channel to the venturi portion are prevented during the removal and attaching of the cap. Alternatively, when the bypass channel is formed outside the housing, the bypass channel is provided with a check valve that prevents backflow flowing from the throat part of the venturi portion.

The sampling device according to the present invention is suitable for uses where a high degree of cleanliness is required strictly, such as sampling of liquid or water used for dialysis.

In the above-described sampling device, it is preferable to adopt a structure having a sensing means monitoring a displacement of the valve member so that the sensing means can sense a status as to whether or not the cap is being attached and/or a status as to whether or not the liquid sampling means (hereinafter, it may also be referred to simply as "sampling means") is being attached. When the cap is not attached to the sampling device, the sampling portion is exposed to the air, and the cleansing of the sampling device by means of the liquid flowing from the bypass channel stops. As a result, there is a fear that the sampling device may be left under a situation where the sampling portion is potentially contaminated. By providing a sensing means capable of sensing a status as to whether or not the cap is being attached so that attaching the cap is not forgotten, it becomes possible to prevent the contamination from occurring, and thus the cleanliness of the sampling flow channel can be maintained more securely. Representative sensing means include, for example, optical sensors, limit switches and reed switches. Additionally, for a method for detecting the displacement of the valve member, it is possible to adopt not only methods of detecting the displacement of the valve member itself directly, but also methods of detecting the displacement of the valve member indirectly by monitoring a displacement of other member working in an interlocking manner (such as a valve member receiver).

### Effect according to the Invention

According to the sampling device of the present invention, because a constant liquid flow occurs in the sampling flow channel by connecting the main flow channel and the throat part of the venturi portion with the sampling device as the cap is attached to the housing, the cap attached makes it possible to prevent the generation of air, the stoppage of liquid and the touch of the air even when the sampling is not performed. Furthermore, because the pressure difference between the throat part of the venturi portion and the sampling flow channel occurs even when the pressure of the liquid flowing in the main flow channel is low, it becomes possible to secure a stable liquid flow that is hardly affected by the changes of the installation or operation conditions of the device, and therefore it becomes possible to realize a sampling device having excellent degree of freedom of installation and operation stability. Furthermore, because the liquid flow occurs in the sampling flow channel without auxiliary power source, the device is prevented from becoming complicated, from upsizing and from increasing in cost. Furthermore, the degree of cleanliness in the sampling flow channel can be maintained more securely by providing a detection means for detecting a state of engagement/disengagement of the cap and/or the sampling means so that forgetfulness of attaching the cap can be monitored.

### Brief explanation of the drawings

[Fig. 1] Fig. 1 is a longitudinal section view of a sampling device according to the first embodiment of the present invention.
[Fig. 2] Fig. 2 is a longitudinal section view of a sampling device according to the second embodiment of the present invention.
[Fig. 3] Fig. 3 is an enlarged longitudinal section view showing vicinities of a valve member in the sampling device of Fig. 2.
[Fig. 4] Fig. 4 is a longitudinal section view of a sampling device according to the third embodiment of the present invention.
[Fig. 5] Fig. 5 is a schematic diagram that depicts a water circulation system for performing an operation test of the sampling device of Fig. 4.
[Fig. 6] Fig. 6 is a longitudinal section view of a sampling device according to the fourth embodiment of the present invention.

### Embodiments for carrying out the Invention

Hereinafter, embodiments of the present invention will be explained referring to figures.

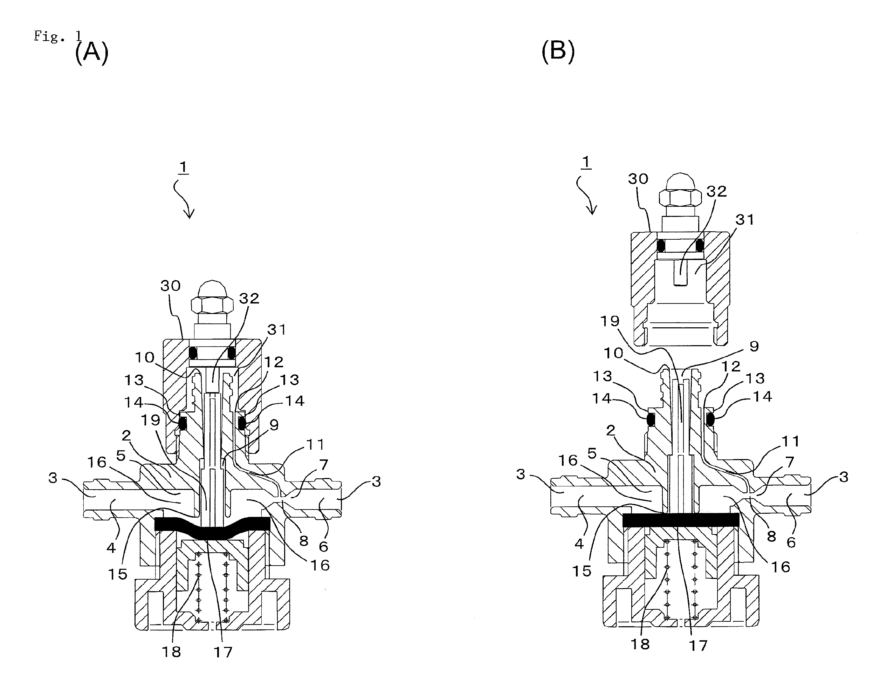

Fig. 1 depicts a sampling device according to the first embodiment of the present invention. In Fig. 1, (A) depicts a situation where a cap is attached to a housing of the sampling device, and (B) depicts a situation where the cap is detached from the housing of the sampling device. In sampling device 1 shown in Fig. 1 (A), housing 2 is provided with main flow channel 3 in which liquid inflow channel 4, branch part 5, and liquid outflow channel 6 having venturi portion 7 are connected in this order. Sampling flow channel 9 and bypass channel 11 are also formed in housing 2. Housing 2 further has sampling flow channel 9 and bypass channel 11. Sampling flow channel 9 branches from branch part 5, and has an opening that opens to the outside of housing 2 when the cap is detached. Bypass channel 11 branches from throat part 8 of venturi portion 7, and opens to the outside of housing 2. Screw thread part 13 is formed on the outer surface of housing 2, and opening end 10 of sampling flow channel 9 and opening 12 of bypass channel 11 are covered with cap 30 being fastened to screw thread part 13 removably. Screw thread part 13 is provided with seal member 14. When cap 30 is attached to screw thread part 13, this seal member 14 comes into tight contact with the inner surface of cap 30 so that inner space 31 of cap 30 is sealed hermetically and liquid leakage is prevented.

Main flow channel side end 15 of sampling flow channel 9 is formed into a shape protruding toward branch part 5, and in branch part 5, ring-shaped flow channel 16 connecting liquid inflow channel 4 with liquid outflow channel 6 is formed around sampling flow channel 9. Furthermore, in branch part 5, valve member 17 that can open and close main flow channel side end 15 of sampling flow channel 9 faces main flow channel side end 15, and is being pushed toward main flow channel side end 15 by spring 18. Liquid inflow channel 4, liquid outflow channel 6 and ring-shaped flow channel 16 are configured not to be blocked by valve member 17. Therefore, even when main flow channel side end 15 of sampling flow channel 9 is being blocked by valve member 17, liquid can flow from liquid inflow channel 4 to liquid outflow channel 6 via ring-shaped flow channel 16 that circumvents sampling flow channel 9.

Movable rod 19 that is slidable along inner surface of sampling flow channel 9 is disposed in sampling flow channel 9, and branch part side end of movable rod 19 comes into contact with valve member 17. The cross section shape of movable rod 19 is approximately in the shape of a cross, and 4 passages being partitioned by movable rod 19 are formed in sampling flow channel 9. However, the shape of movable rod 19 is not particularly limited to the above-described embodiment as long as liquid can flow through sampling channel 9 in a situation where movable rod 19 is disposed in sampling channel 9.

Cap 30 is provided with pin 32. When cap 30 is screwed to screw thread part 13 of housing 2, movable rod 19 that is pushed by pin 32 pushes valve member 17 against spring 18, sampling flow channel 9 is opened, and sampling flow channel 9 becomes in fluid communication with main flow channel 3.

As shown in Fig. 1 (A), when cap 30 is attached screw thread part 13 of housing 2 and sampling flow channel 9 is opened, the greater part of the liquid flowing into liquid inflow channel 4 circumvents sampling flow channel 9 by flowing through ring-shaped flow channel 16 in branch part 5, goes through venturi portion 7 of liquid outflow channel 6, and flows from the end of liquid outflow channel 6 to the subsequent flow channel. In a situation where liquid passes through main flow channel 3, while the liquid flows through venturi portion 7, throat part 8 that is a narrowed portion in venturi portion 7 accelerates the liquid flowing therein and causes a reduction in pressure. On the other hand, the rest of liquid flowing into liquid inflow channel 4 enters sampling flow channel 9 at branch part 5 via main flow channel side end 15, flows through inner space 31 of cap 30 and bypass channel 11, and joins the majority of liquid that flows in main flow channel 3 at throat part 8 of venturi portion 7. As described above, the liquid flowing through main flow channel 3 generates a relatively negative pressure, which is caused by the above-described pressure reduction, at throat part 8 of venturi portion 7. A continuous liquid flow is generated in a route from sampling flow channel 9 via bypass channel 11 to throat part 8 due to a suction force caused by the pressure difference between main flow channel and the route, and therefore liquid stagnancy and air stagnancy are prevented in this route. Due to the continuous liquid flow in sampling flow channel 9, inner space 31 of cap 30 and bypass channel 11, it becomes possible in these flow channels to inhibit the growth of bacteria and accumulation of ingredients in liquid, and thereby to maintain the degree of cleanliness appropriately.

When sampling liquid, a sampling means, e.g. a syringe (not depicted in Figures), is attached to opening end 10 of sampling flow channel 9 after cap 30 is detached from screw thread part 13 of housing 2, so that a sample of liquid can be obtained from sampling flow channel 9. When cap 30 is detached from screw thread part 13 and movable rod 19 is released from being pushed by pin 32, as shown in Fig. 1(B), valve member 17 closes main flow channel side end 15 of sampling flow channel 9 by an action of spring 18. Due to this closure sampling flow channel 9 is cut off from main flow channel 3, and effusion of liquid from opening end 10 is prevented. On the other hand, because the fluid communication between liquid inflow channel 4 and liquid outflow channel 6 is secured by ring-shaped flow channel 16 disposed at branch part 5, liquid can continue to flow from liquid inflow channel 4 to liquid outflow channel 6, even in a situation where cap 30 is detached and sampling flow channel 9 is cut off from main flow channel 3.

When the sampling means, e.g. a syringe, is attached to opening end 10 of sampling flow channel 9 and pushes moving rod 19 either concurrently or after being attached, valve member 17 is pushed down against spring 18 and the closure of sampling flow channel 9 by means of valve member 17 is released. As a result, part of liquid flowing through main flow channel 3 enters sampling flow channel 9 from main flow channel side end 15 and is stored in the syringe.

When liquid sampling is finished and the syringe is removed, valve member 17 is pushed toward main flow channel side end 15 of sampling flow channel 9 by means of spring 18, sampling flow channel 9 is cut off from main flow channel 3 once again, and effusion of liquid from opening end 10 is prevented.

When cap 30 is attached to screw thread part 13 of housing 2 as shown in Fig. 1 (A), moving rod 19 being pushed by pin 32 pushes valve member 17 down against spring 18, the closure of sampling flow channel 9 is released, and part of liquid flowing through main flow channel 3 enters sampling flow channel 9. Because the liquid flow causes a negative pressure at throat part 8 of venturi portion 7 as described above, a continuous liquid flow is generated in the route from sampling flow channel 9 through bypass channel 11 to throat part 8 due to the pressure differential, therefore liquid stagnancy and air stagnancy are prevented and the degree of cleanliness is maintained appropriately in these flow channels.

Moreover, as with the normal case above, the configuration of sampling device 1 also functions effectively in the case where a cleansing liquid instead of the liquid is fed in the whole liquid processing system including sampling device 1 so that the whole liquid processing system is cleansed. By performing the above-described cleansing process under the situation where cap 30 is equipped with housing 2 of sampling device 1, part of the cleansing liquid flowing through main flow channel 3 enters sampling flow channel 9, and therefore the cleansing liquid flows through sampling flow channel 9, inner space 31 of cap 30 and bypass channel 11 as well as main flow channel 3. When the cleansing liquid flows in main flow channel 3 and sampling flow channel 9, because of the pressure difference a flow of the cleansing liquid is generated in the route from sampling flow channel 9 through bypass channel 11 to throat part 8 of venturi portion 7. As a result, it becomes possible to cleanse sampling flow channel 9 quickly and efficiently, and it also becomes possible to reduce the cleansing time and to improve the degree of cleanliness.

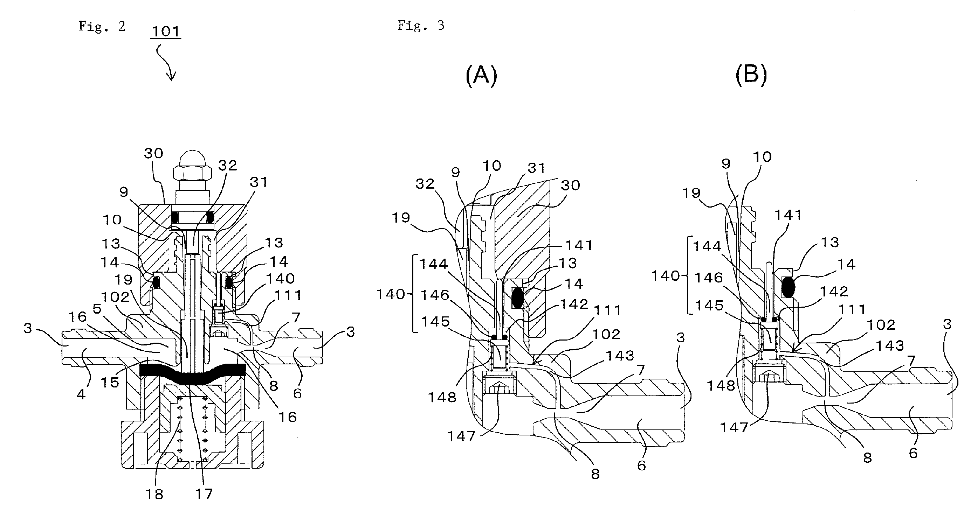

Fig. 2 and Fig. 3 depict a sampling device according to the second embodiment of the present invention, and more specifically, depict an example wherein a bypass channel is formed inside the housing and a valve member, which closes the bypass channel when a cap is detached from the housing and which stops closing the bypass channel when the cap is attached to the housing, is provided in the bypass channel. In sampling device 101 of Fig. 2, housing 102 is provided with main flow channel 3 wherein liquid inflow channel 4, branch part 5, and liquid outflow channel 6 that has venturi portion 7 communicate with each other in this order. In housing 102, sampling flow channel 9 and bypass channel 111 are also provided. Sampling flow channel 9 having an opening to the outside of housing 2 branches from branched part 5, and bypass channel 111 having an opening to the outside of housing 2 branches from throat part 8 of venturi portion. Additionally, valve part 140 that can open/close bypass channel 111 is provided in bypass channel 111. Screw thread part 13 is formed on the surface of housing 102, and opening end 10 of sampling channel 9 and opening end of bypass channel 111 are covered with cap 30 being attached to screw thread part 13 removably. Sealing member 14 is mounted on screw thread part 13. When cap 30 is attached to screw thread part 13, seal member 14 comes into tight contact with the inner surface of cap 30 so that inner space 31 of cap 30 is sealed hermetically to prevent liquid leakage.

Fig. 3 is an enlarged cross-sectional view that shows an enlarged view around valve part 140 of sampling device 101 depicted in Fig. 2. Fig. 3 (A) depicts a situation where cap 30 is being attached to housing 102, and Fig. 3 (B) depicts a situation where cap 30 is being detached from housing 102. In Fig. 3 (A), bypass channel 111 comprises inflow port 141 having an opening to the outside of housing 102, valve chamber 142 having an opening to branch part 5, and outflow port 143 having an opening to throat part 8 of venturi portion 7 that is provided in liquid outflow channel 6. Additionally, valve part 140 being slidable along an axial direction of inflow port 141 is provided in bypass channel 111, and is comprised of shaft part 144 being inserted in inflow port 141 movably, base part 145 being accommodated in valve chamber 142, and seal part 146 being capable of closing inflow port 141. The opening of inflow port 141 of bypass channel 111 is covered with cap 30 that is attached to screw thread part of housing 102, and shaft part 144 of valve part 140 is being pushed toward valve chamber by cap 30. Valve chamber 142 accommodates spring 148 that is supported by setscrew 147 sealing the opening of valve chamber 142 toward branch part 5. Valve part 140 is being pushed toward inflow port 141 by spring 148. The other portions of sampling device 101 are configured in a manner similar to sampling device 1 depicted in Fig. 1 (A), therefore the explanation thereof will be omitted by assigning the same symbols as Fig. 1 (A).

When cap 30 is attached to screw thread part 13 of housing 102 in sampling device 101, the closure of sampling flow channel 9 and bypass channel 111 becomes released. As shown in Fig. 3 (A), when cap 30 pushes shaft part 144 of valve part 140 against spring 148, valve part 140 is pushed down toward spring 148 and the closure of inflow port 141 caused by seal part 146 of valve part 140 becomes released. On the other hand, in sampling flow channel 9, as shown in Fig. 2, because moving rod 19 being pushed by pin 32 of cap 30 pushes valve member 17 against spring 18, the closure of main flow channel side end 15 of sampling flow channel 9 becomes released and sampling flow channel 9 fluidly communicates with main flow channel 3. As a result, a route in which main flow channel side end 15 of sampling flow channel 9, sampling flow channel 9, inner space 31 of cap 30, inflow port 141, valve chamber 142, outflow port 143 and throat part 8 of venturi portion 7 fluidly communicate in this order is formed.

When cap 30 is attached to screw thread part 13 of housing 2, the greater portion of liquid that enters liquid inflow channel 4 flows ring-shaped flow channel 16 in branch part 5, passes through venturi portion 7 of liquid outflow channel 6 and flows from the end of liquid outflow channel 6 to the subsequent flow channel. This liquid flow in main flow channel 3 causes a relatively negative pressure in throat part 8 of venturi portion 7. On the other hand, the rest portion of liquid that enters liquid flow channel 4 passes main flow channel side end 15 in branch part 5, enters sampling flow channel 9, flows inner space 31 of cap 30, passes through bypass channel 111 including inflow port 141, valve chamber 142 and outflow port 143, and joins liquid that flows through main flow channel 3 in throat part 8 of venturi portion 7. The suction force caused by the pressure differential from main flow channel generates a continuous liquid flow in the fluid path from sampling flow channel 9 via bypass channel 111 to throat part 8. In this fluid path, therefore, liquid stagnancy and air stagnancy are prevented, multiplication of bacteria and accumulation of liquid components are suppressed, and the degree of cleanliness is maintained appropriately.

On the other hand, when cap 30 is detached from screw thread part 13 of housing 102, both sampling flow channel 9 and bypass channel 11 are closed. As shown Fig. 3 (B), in bypass channel 111, valve part 140 is pushed by spring 148 toward inflow port 141, seal part 146 of valve part 140 comes into tight contact with the surrounding portion of an opening of inflow port 141 that opens to valve chamber 142. As a result, the inflow of air from inflow port 141 of bypass 111 to venturi portion 7 is cut off, the intrusion of dust and bacteria in the air is prevented, and the degree of cleanliness of bypass channel 111 as well as main flow channel 3 is maintained even when the cap 30 is detached. Furthermore, in sampling flow channel 9, moving rod 19 stopped pushing as cap 30 is detached, valve member 17 being pushed by spring 18 closes main flow channel side end 15 of sampling flow channel 9 from main flow channel 3, and consequently liquid leakage from open end 10 is prevented. On the other hand, because the fluid communication between liquid inflow channel 4 and liquid outflow channel 6 is secured by ring-shaped flow channel 16 disposed at branch part 5, liquid can continue to flow from liquid inflow channel 4 to liquid outflow channel 6, even in a situation where cap 30 is detached and sampling flow channel 9 is closed from main flow channel 3.

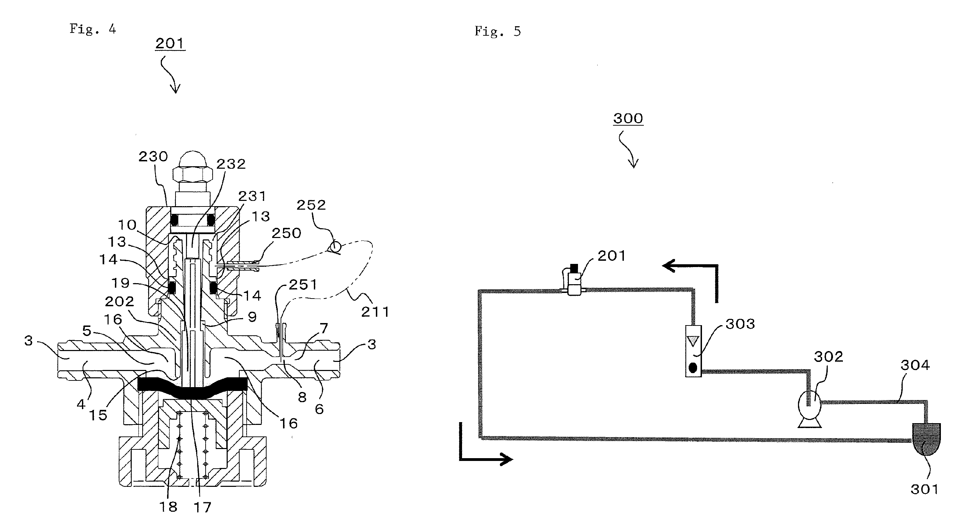

Fig. 4 depicts a sampling device according to the third embodiment of the present invention, and more specifically, shows an example wherein a bypass channel is disposed outside the housing and a check valve that prevents backflow coming from the throat part of the venturi portion is disposed in the bypass channel. In sampling device 201 of Fig. 4, housing 202 is provided with main flow channel 3 in which liquid inflow channel 4, branch part 5, and liquid outflow channel 6 that has venturi portion 7 are connected in this order. Housing 202 further has sampling flow channel 9 that branches from branch portion 7 and has an opening to the outside of housing 202. Screw thread part 13 is formed on the outer surface of housing 202, and open end 10 of sampling 9 is covered with cap 230 that is removably attached to screw thread part 13. Sealing member 14 is mounted on screw thread part 13. When cap 230 is attached to screw thread part 13, seal member 14 comes into tight contact with the inner surface of cap 230, and the hermeticity of inner space 31 of cap 30 is secured so that liquid leakage is prevented. Cap 230 has pin 232, which pushes moving rod 19 in sampling flow channel 9 toward valve member 17 when cap 230 is attached, and is provided with outlet port 250 connecting inner space 231 of cap 230 to the outside. Bypass channel 211 that connects outlet port 250 to outlet port 251 of housing 202 is disposed outside the housing. Check valve 252 is disposed in bypass channel 211 so that backflow from throat part 8 of venturi portion 7 is prevented. The other portions of sampling device 201 are configured in a manner similar to sampling device 1 depicted in Fig. 1 (A), therefore the explanation thereof will be omitted by assigning the same symbols as Fig. 1 (A).

When cap 230 is screwed to screw thread part 13 of housing 202, movable rod 19 is pushed toward valve member 17 by pin 232 of cap 230, and valve member 17 is pushed against spring 18. As a result, the blockage of main flow channel side end 15 of sampling flow channel caused by valve member 17 is cleared, and sampling flow channel 9 comes in fluid communication with main flow channel 3. The greater part of liquid that enters liquid inflow channel 4 flows to liquid outflow channel 6 via ring-shaped flow channel 16 and venturi portion 7, and due to the pressure reduction that is caused by this flow, a relatively negative pressure is generated in throat part 8 of venturi portion 7. On the other hand, the rest part of liquid that enters liquid inflow channel 4 flows to sampling flow channel 9 via main flow channel side end 15 in branch portion 5, passes from inner space 231 of cap 230 through outlet port 250 to bypass channel 211, flows from outlet port 251 to throat part 8 of venturi portion 7, and joins the liquid flowing in main flow channel 3. The suction force caused by the pressure differential from main flow channel generates a continuous liquid flow in the fluid path from sampling flow channel 9 to throat part 8 via bypass channel 211. In this fluid path, therefore, liquid stagnancy and air stagnancy are prevented, multiplication of bacteria and accumulation of liquid components are suppressed, and the degree of cleanliness is maintained appropriately.

When cap 230 is detached from screw thread part 13 of housing 202, pin 232 of cap 230 no longer pushes movable rod 19, valve member 17 is pushed toward main flow channel side end 15 of sampling flow channel 9 by spring 18, and sampling flow channel is shut from main flow channel 3. On the other hand, because the fluid communication between liquid inflow channel 4 and liquid outflow channel 6 is secured by ring-shaped flow channel 16 disposed at branch portion 5, liquid can continue to flow from liquid inflow channel 4 to liquid outflow channel 6, even in a situation where cap 230 is detached and sampling flow channel 9 is shut from main flow channel 3.

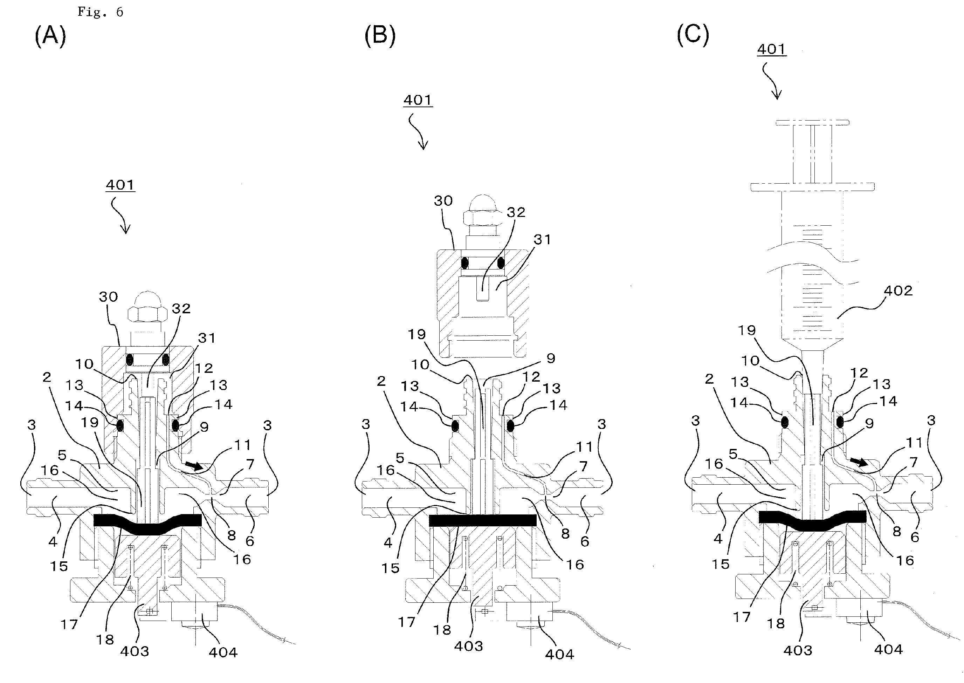

Fig. 6 shows sampling device 401 according to the fourth embodiment of the present invention. In Fig. 6, (A) depicts a situation where cap 30 is attached to sampling device 401, (B) depicts a situation where cap 30 is detached from sampling device 401, and (C) depicts a situation where syringe 402 as a liquid sampling means is attached to sampling device 401. Furthermore, sampling device 401 is provided with optical sensor 404 as a detecting means that detects a displacement of valve member 17 in accordance with a displacement of valve member receiver 403. The other portions of sampling device 401 are configured in a manner similar to sampling device 1 depicted in Fig. 1 (A), therefore the explanation thereof will be omitted by assigning the same symbols as Fig. 1 (A).

Cap 30 has pin 32, and when cap 30 is attached to housing 2 as shown in Fig. 6 (A), movable rod 19 that is pushed by pin 32 pushes down valve member 17 and valve member receiver 403 against spring 18, and valve member 17 stops shutting sampling flow channel 9 from main flow channel 3. Furthermore, optical sensor 404 detects the displacement of valve member receiver 403 and provides a notification that the blockage of sampling flow channel caused by valve member 17 is cleared, that is, a notification that the cap is attached.

When cap 30 is detached from housing 2, pin 32 is removed from pushing movable rod 19, and spring 18 pushes valve member receiver 403 and valve member 17 toward main flow channel side end 15 of sampling flow channel 9. As a result, main flow channel side end 15 of sampling flow channel 9 is blocked by valve member 17 as shown in Fig. 6 (B), and sampling flow channel 9 is shut from main flow channel 3. Furthermore, because such displacements of valve member receiver 403 and valve member 17 are detected by optical sensor 404, users can notice that cap 30 has been detached from sampling device 401.

When liquid sampling is performed, cap 30 is detached from housing 2, syringe 402 as a sampling means is attached to open end 10 of sampling flow channel 9, and liquid is collected from sampling flow channel 9. When syringe 402 is attached to opening end 10 of sampling flow channel 9 and pushes moving rod 19 either concurrently or after being attached, valve member 17 and valve member receiver 403 are pushed down against spring 18, and the blockage of sampling flow channel 9 by means of valve member 17 is cleared. When movable rod 19 is pushed down by pushing syringe 402 either concurrently or after attaching syringe 402 to opening end 10 of sampling flow channel 9, as shown in Fig. 6 (C), valve member 17 and valve member receiver 403 are pushed down against spring 18, and the blockage of sampling flow channel 9 by means of valve member 17 becomes cleared. In this case, because the displacements of valve member receiver 403 and valve member 17 are detected by optical sensor 404, users can confirm whether syringe 402 is attached securely or not.

When liquid sampling is finished and syringe 402 is removed, as shown in Fig. 6 (B), valve member 17 is pushed toward main flow channel side end 15 of sampling flow channel 9 by spring 18 via valve member receiver 403. As a result, sampling flow channel 9 is shut from main flow channel once again, liquid leakage from opening end 10 is prevented, and optical sensor 404 detects the shut-off of sampling flow channel 9 and the removal of syringe 402.

According to sampling device 401 shown in Fig. 6, due to optical sensor 404 it becomes possible to detect whether cap 30 is attached or not and whether syringe 402 is attached or not. Therefore, it becomes possible to monitor a failure that cap 30 is forgotten to be attached, and the degree of cleanliness in sampling flow channel 9 can be maintained more securely. The detecting means for detecting the displacement of valve member 17 are not limited to optical sensors particularly, and include various detecting means such as limit switches and reed switches. Moreover, a means other than a syringe can also be used as the sampling means.

### Examples.

Using liquid circulation system 300 shown in Fig. 5, an operation test of sampling device 201 shown in Fig. 4 has been performed. In liquid circulation system 300 of Fig. 5, pump 302 that makes water from water tank 301 circulate through the system, flowmeter 303 and sampling device 201 are disposed in this order along the water flow direction (arrowed direction in Fig. 5). In venturi portion 7 of sampling device 201, liquid outflow channel 6 having a diameter of 4 mm (a cross sectional area of flow is 12.56 mm²) is provided with throat part 8 having a diameter of approximately 2.8 mm (a cross sectional area of flow is approximately at half thereof). As for bypass channel 211 of sampling device 201 and pipes 304 that connect devices, transparent tubes are used so that it becomes possible to observe inside the tubes. As for pump 302 and flowmeter 303, the following devices are used.
Pump: IWAKI Magnet Gear Pump MDG-R2BB24-03A
Flowmeter: TOKYO KEISO flowmeter capable of controlling the rate of flow (range: 200-700 ml/min)

### (Experiment 1)

Under the situation where cap 30 is attached to sampling device 201, colorless water is circulated through water circulation system 300, and it is confirmed visually that water circulates through water circulation system 300 properly without leakage.

### (Experiment 2)

Under the situation where cap 230 is attached to sampling device 201, an operation test is performed by circulating colored water through water circulation system 300. The experiment is performed under 2 conditions of a flow rate of 200 ml/min and a flow rate of 700 ml/min, and in each case it is confirmed visually that colored water flows through bypass channel 211 of sampling device 201.

### (Experiment 3)

Under the situation where cap 230 is attached to sampling device 201, colored water is circulated through water circulation system 300 at a flow rate of 200 ml/min. During the circulation bypass channel 211 is detached from outlet port 251, and by observing the air entering from outlet port 251 toward the downstream direction of venturi portion 7, it is confirmed that a suction force occurs in outlet port 251 toward throat part 8 of venturi portion 7.

### (Experiment 4)

Under the situation where cap 230 is attached to sampling device 201, check valve 252 that is disposed in bypass channel 211 is replaced with a flowmeter (not shown). After the replacement, water is circulated through water circulation system 300 at a flow rate of 500 ml/min, and it is confirmed that the flowmeter detects a flow.

### (Experiment 5)

Under the situation where cap 230 is attached to 201 after the flowmeter is replaced with check valve 252 in bypass channel 211, water is circulated through water circulation system 300 at a flow rate of 500 ml/min. Then cap 230 is detached during the circulation, and it is confirmed that there is no water leakage at either opening end 10 of sampling flow channel 9 or cap 230.

### Industrial Applications of the Invention

The sampling device according to the present invention is particularly suitable as a sampling device used in a dialysis system where high degree of cleanliness is required.

### Explanation of symbols

- 1, 101, 201, 401:: sampling device
- 2, 102, 202:: housing
- 3:: main flow channel
- 4:: liquid inflow channel
- 5:: branch part
- 6:: liquid outflow channel
- 7:: venturi portion
- 9:: sampling flow channel
- 10:: opening end
- 11:: Bypass channel
- 12:: opening
- 13:: screw thread part
- 14:: seal member
- 15:: main flow channel side end
- 16:: ring-shaped flow channel
- 17:: valve member
- 18:: spring
- 19:: movable rod
- 30, 230:: cap
- 31, 231:: inner space
- 32, 232:: pin
- 140:: valve part
- 141:: inflow port
- 142:: valve chamber
- 143:: outflow port
- 144:: shaft part
- 145:: base part
- 146:: seal part
- 147:: setscrew
- 148:: spring
- 250:: outlet port
- 251:: inlet port
- 252:: check valve
- 300:: liquid circulation system
- 301:: water tank
- 302:: pump
- 303:: flowmeter
- 304:: pipe
- 402:: syringe
- 403:: valve member receiver
- 404:: optical sensor

## Claims

1. A sampling device, comprising:
a housing having a main flow channel including a liquid inflow channel and a liquid outflow channel, said housing further having a sampling flow channel branching from a branching part provided between said liquid inflow channel and said liquid outflow channel, one end of said sampling channel being formed as an open end;
a cap for covering said open end part of said sampling flow channel, said cap being detachable from and attachable to said housing; and
a valve provided in an urged state so as to interrupt said sampling flow channel from said main flow channel, said valve being configured to release the interruption of said sampling flow channel from said main flow channel when attaching said cap to said housing or when attaching a liquid sampling means to said open end part of said sampling channel subsequent to detaching said cap from said housing;
wherein a venturi portion is provided to said liquid outflow channel; and
wherein said sampling device is provided with a bypass channel being configured to connect an inner space of said cap to said throat part of said venturi portion when said cap is attached to said housing.

2. The sampling device according to claim 1, wherein said bypass channel is formed within said housing.

3. The sampling device according to claim 2, wherein said sampling device is provided with a valve that shuts the bypass channel when the cap is detached from the housing and that stops shutting the bypass channel when the cap is attached to the housing.

4. The sampling device according to claim 1, wherein said bypass channel is formed outside said housing.

5. The sampling device according to claim 4, wherein a check valve that prevents backflow flowing from said throat part of said venturi portion is provided in said bypass channel.

6. The sampling device according to any of claims 1-5, wherein said liquid is dialysis liquid or dialysis water.

7. The sampling device according to any of claims 1-6, wherein said sampling device has a sensing means monitoring a displacement of said valve member so that said sensing means can sense a status as to whether or not said cap is being attached and/or a status as to whether or not said liquid sampling means is being attached.
